# FASCICULE DE BREVET EUROPEEN

(11) **EP 2 448 902 B1**
(45) Date de publication et mention de la délivrance du brevet: **30.07.2014**
(21) Numéro de dépôt: 10730138.4
(22) Date de dépôt: 28.06.2010
(51) Int. Cl.: C07C 51/09, C07C 67/03

(54) **PROCEDE DE PREPARATION DE L'ACIDE DIFLUOROACETIQUE**
VERFAHREN ZUR HERSTELLUNG VON DIFLUORESSIGSÄURE
METHOD FOR PREPARING DIFLUOROACETIC ACID

(30) Priorité: 30.06.2009 FR 0903184
(43) Date de publication de la demande: 09.05.2012
(73) Titulaire: Rhodia Opérations, 75009 Paris (FR)
(72) Inventeur: BUISINE, Olivier, 69230 Saint-genis Laval (FR); CHIOVATO, Alessandro, 69480 Anse (FR)
(74) Mandataire: Blanchard, Isabelle Jackie
(86) Numéro de dépôt international: PCT/EP2010/059142
(87) Numéro de publication internationale: WO 2011/000804

(56) Documents cités:
- GRYSZKIEWICZ-TROCHIMOWSKI M M E ET AL: "RECHERCHES SUR LES COMPOSES ORGANIAQUES FLUORES DANS LA SERIE ALIPHATIQUE. II. SUR LES DERIVES DES ACIDES MONO-, DI- ET TRI-FLUORO-ACETIQUES" RECUEIL DES TRAVAUX CHIMIQUES DES PAYS-BAS, ELSEVIER SCIENCE PUBLISHERS, AMSTERDAM, NL, vol. 66, 1 janvier 1947 (1947-01-01), pages 419-426, XP001194708 ISSN: 0165-0513
- DATABASE WPI Week 200234 7 février 2002 (2002-02-07) Thomson Scientific, London, GB; AN 2002-303929 XP002567490 & WO 02/10108 A1 (ASAHI GLASS CO LTD) 7 février 2002 (2002-02-07)
- COHN, BERGMANN: ISRAEL JOURNAL OF CHEMISTRY, vol. 2, no. 6, 18 novembre 1964 (1964-11-18), pages 355-361, XP009129231

## Description

La présente invention a pour objet un nouveau procédé de préparation de l'acide difluoroacétique.

L'invention vise la préparation d'un acide difluoroacétique de haute pureté.

Il existe dans la littérature plusieurs voies d'accès à l'acide difluoroacétique.

L'une des voies d'accès décrite dans EP-A 1 137 615 consiste à effectuer l'hydrogénodéshalogénation de l'acide chlorodifluoroacétique, par hydrogénation en milieu basique et en présence notamment de nickel de Raney.

L'acide difluoroacétique peut être préparé également à partir de l'acide chlorodifluoroacétique, selon un procédé en phase gazeuse comme décrit dans US 5 455 376 : l'hydrogénation étant conduite entre 120°C et 250°C, en présence d'un catalyeur à base de palladium/platine et/ou nickel sous forme métallique ou supportée, par exemple sur alumine.

Il est connu également selon JP-A 6228043 de préparer l'acide difluoroacétique par hydrolyse basique d'un N,N-dialkyldifluoroacétamide : ce dernier étant préparé par échange KF d'un N,N-dialkyldichloroacétamide obtenu par réaction du chlorure de dichloroacétyle et d'une dialkylamine.

E. Gryszkiewicz-Trochimowski et al (Recueil des Travaux Chimiques des Pays-Bas, 1947, 66, 419-426) décrivent la formation de l'acide difluoroacétique par saponification de l'ester méthylique de l'acide difluoroacétique, en présence d'eau et de chaux.

Tous ces procédés conduisent à l'acide difluoroacétique ou son sel.

Le problème qui se pose est qu'il est très difficile de purifier l'acide difluoroacétique obtenu en solution aqueuse, car c'est un produit qui est soluble dans l'eau et dans ces conditions, plus difficilement séparable par distillation.

Par ailleurs, il est décrit dans WO 02/10108 un procédé de préparation d'un ester fluoré R^{d}-O-CO-R^{cf} et d'un alcool fluoré R^{af}R^{bf}CHOH selon une réaction de transestérification classique d'un ester fluoré R^{af}R^{bf}CH-O-CO-R^{cf} avec un alcool R^{d}OH ; l'alcool formé étant distillé au fur et à mesure de sa formation.

H. Cohn et al (Israël Journal of Chemistry 2, 1964, 355-361) décrivent la préparation d'acides perhalogénés (acide bromodifluoroacétique, acide dibromofluoroacétique) par transestérification des esters éthyliques correspondants avec l'acide formique ; le formiate d'éthyle étant distillé au fur et à mesure. Toutefois, l'enseignement ne concerne que les acides perhalogénés.

L'objectif de la présente invention est de fournir un procédé simple de préparation de l'acide difluoroacétique, permettant de l'obtenir pur.

Par « haute pureté », on entend dans le présent texte, un acide difluoroacétique ayant une pureté supérieure ou égale à 95 % en poids, de préférence supérieure ou égale à 98 % et plus préférentiellement supérieure ou égale à 99 %.

Il a maintenant été trouvé et c'est ce qui constitue l'objet de la présente invention, un procédé de préparation de l'acide difluoroacétique caractérisé par le fait qu'il comprend :
- la réaction d'un ester de l'acide difluoroacétique avec un acide aliphatique carboxylique conduisant, suite à une réaction de transestérification, à la formation de l'acide difluoroacétique et de l'ester de l'acide carboxylique correspondant ; l'acide carboxylique étant choisi de telle sorte que l'ester dudit acide carboxylique ait un point d'ébullition inférieur à celui de l'acide difluoroacétique,
- l'élimination par distillation de l'ester dudit acide carboxylique au fur et à mesure de sa formation permettant ainsi de récupérer l'acide difluoroacétique.

On donne ci-après, le schéma réactionnel du procédé de l'invention pour faciliter la compréhension de l'invention sans pour autant lier la portée de l'invention, à celui-ci.

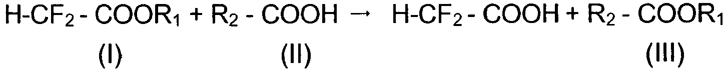

Conformément au procédé de l'invention, on obtient un acide difluoroacétique de haute pureté d'au moins 95 % ce qui implique que des opérations de purification subséquentes sont possibles mais pas nécessaires.

Cette bonne pureté résulte du fait que le taux de transformation de l'ester de l'acide difluoroacétique de formule (I) est quasi-total et que le produit coproduit par la réactions, à savoir l'ester de l'acide carboxylique de formule (III) est éliminé au fur et à mesure de sa formation.

L'acide difluoroacétique obtenu selon le procédé de l'invention est anhydre avec une teneur en eau avantageusement inférieure à 1 % en poids.

Intervient donc dans le procédé de l'invention, un ester de l'acide difluoroacétique dont le groupe hydrocarboné R₁ de la fonction ester COOR₁ est déterminé de telle sorte qu'il conduise à un ester de l'acide carboxylique (III) qui présente un point d'ébullition inférieur à celui de l'acide difluoroacétique.

Ce groupe R₁ est avantageusement un groupe alkyle ayant une faible condensation en carbone, par exemple de 1 à 4 atomes de carbone mais l'on préfère que R₁ représente un groupe méthyle, éthyle, propyle ou isopropyle.

Ainsi, préférentiellement, les esters de l'acide difluoroacétique répondent à la formule suivante :

H-CF₂-COOR₁ (I)

dans ladite formule :
- R₁ représente un groupe alkyle ayant de 1 à 4 atomes de carbone, de préférence 1 ou 2 atomes de carbone.

Les composés de formule (I) préférentiellement mis en oeuvre dans le procédé de l'invention sont des esters d'alkyle, de préférence l'ester méthylique ou éthylique de l'acide difluoroacétique.

Les esters de l'acide difluoroacétique de formule (I) sont des composés disponibles dans le commerce ou pouvant aisément être préparés selon les procédés décrits dans la littérature.

Une voie d'accès privilégiée consiste à faire réagir un alcool R₁OH avec un fluorure de difluoroacétyle de formule H-CF₂ - COF. On peut se référer en particulier à la préparation décrite dans EP-A 0 694 523.

Pour ce qui est de la source de protons permettant d'effectuer la transestérification, on la symbolise par la formule (II), R₂ - COOH dont le groupe hydrocarboné R₂ lié à la fonction acide COOH est déterminé de telle sorte qu'il conduise à un ester de l'acide carboxylique (III) qui présente un point d'ébullition inférieur à celui de l'acide difluoroacétique.

Les acides aliphatiques carboxyliques choisis préférentiellement pour être mis en oeuvre dans le procédé de l'invention répondent à la formule suivante :

R₂ - COOH (II)

dans ladite formule :
- R₂ représente un atome d'hydrogène, un groupe alkyle ayant 1 ou 2 atomes de carbone, un groupe halogénoalkyle ayant 1 atome de carbone dont un à 3 atomes d'hydrogène peuvent être remplacés par un atome d'halogène.

Par « atome d'halogène », on entend un atome de fluor, chlore ou brome.

On fait appel plus particulièrement, à titre de composés de formule (II), à l'acide formique, l'acide acétique, l'acide trifluoroacétique, l'acide monochloroacétique, l'acide dichloroacétique, l'acide trichloroacétique.

Parmi les composés précités, l'acide formique est préféré.

Conformément à l'invention, on fait réagir l'ester de l'acide difluoroacétique de formule (I) et l'acide aliphatique carboxylique de formule (II), dans des proportions telles que le rapport entre le nombre de moles d'ester d'acide difluoroacétique et le nombre de moles de l'acide aliphatique carboxylique varie entre 0,8 et 1,2, et est égal de préférence à 1.

La réaction de transestérification est préférentiellement conduite en l'absence de catalyseur mais l'invention n'exclut pas la mise en oeuvre d'un catalyseur classiquement utilisé pour ce type de réaction, à savoir un catalyseur de type acide fort. On peut citer notamment, l'acide sulfurique, l'acide p-toluène sulfonique,

Comme autres exemples de catalyseurs acides protiques convenant également, on peut citer les résines constituées d'un squelette polystyrénique qui porte des groupes sulfoniques et plus particulièrement les résines commercialisées sous différentes dénominations commerciales : AMBERLYST 15, AMBERLYST 35, AMBERLYST 36 ou les résines perfluorées porteuses de groupes sulfoniques et, plus particulièrement le NAFION qui est un copolymère de tétrafluoroéthylène et de perfluoro[2-(fluorosulfonyléthoxy)-propyl] vinyl éther.

La quantité de catalyseur mis en oeuvre, exprimée en protons H⁺ par rapport à l'ester de l'acide difluoroacétique de formule (I), représente généralement de 0,1 à 10 % en moles.

Le procédé, objet de la présente invention comprend au moins une étape de mélange de l'ester de l'acide difluoroacétique de formule (I) et de l'acide aliphatique carboxylique de formule (II) et une étape de distillation conçue pour obtenir :
- en pied de distillation, l'acide difluoroacétique attendu,
- et en tête de distillation, une phase gazeuse comprenant l'ester formé de formule (III).

Un premier mode de réalisation de l'invention consiste à conduire le procédé de l'invention en discontinu.

Le procédé de l'invention comprend une opération de mélange de l'ester de l'acide difluoroacétique de formule (I) et de l'acide aliphatique carboxylique de formule (II) par mise en contact des deux réactifs de préférence à température ambiante (le plus souvent entre 15°C et 25°C) puis l'on porte le mélange réactionnel maintenu sous pression atmosphérique, à une température telle que l'on distille l'ester de l'acide carboxylique de formule (III) au fur et à mesure de sa formation.

On distille le mélange en recueillant en tête de distillation l'ester de formule (III), éventuellement l'un des deux réactifs en excès et en pied de distillation, l'acide difluoroacétique.

On introduit les réactifs séparément ou en mélange dans un réacteur équipé de moyens d'agitation et de chauffage et surmonté d'une colonne de distillation.

Pour effectuer la distillation, l'apport des calories en pied de colonne peut être fait notamment par circulation d'un fluide caloporteur dans la double enveloppe d'un réacteur ou un autre mode consiste à chauffer dans un échangeur thermique, une fraction du mélange réactionnel prélevé en pied de distillation : le flux prélevé traverse de bas en haut, un échangeur thermique et en sortie d'échangeur est introduit latéralement dans la partie inférieure de la colonne de distillation. La circulation du flux peut être naturelle ou forcée à l'aide d'une pompe.

L'opération de distillation vise à obtenir en pied l'acide difluoroacétique dépourvu de l'ester de formule (III) qui est éliminé en tête de distillation.

Dans le cas où, l'un des réactifs est en excès, il est distillé après distillation de l'ester de formule (III).

La distillation de l'ester de formule (III) est conduite à une température dans le bouilleur choisie de manière que la température soit suffisante pour obtenir en tête de colonne, la température d'ébullition de l'ester de formule (III).

On conduit de préférence la distillation sous pression atmosphérique. Toutefois, une pression légèrement inférieure ou supérieure à la pression atmosphérique est également possible.

Dans le cas préféré de la mise en oeuvre de l'acide formique ou trifluoroacétique, comme acide carboxylique, la température dans le bouilleur reste inférieure à 100°C, sous pression atmosphérique et se situe de préférence entre 50 et 100°C

La distillation est effectuée à l'aide d'un colonne de distillation classique.

L'homme du métier est parfaitement en mesure de choisir les moyens à mettre en oeuvre en fonction des composés à séparer.

On rappellera simplement ce qui suit. La taille (notamment le diamètre) des colonnes de distillation dépend du flux circulant et de la pression interne. Leur dimensionnement se fera donc principalement suivant le débit de mélange à traiter. Le paramètre interne qu'est le nombre d'étages théoriques est déterminé notamment par la pureté du composé de départ et la pureté du produit devant être obtenus en tête de distillation.

On précisera que les colonnes pourront être garnies indifféremment de plateaux ou de garnissage ordonné, comme cela est parfaitement connu de l'homme du métier.

L'installation étant déterminée, l'homme du métier ajuste les paramètres de fonctionnement de la colonne.

Ainsi, la colonne de distillation pourra être avantageusement, mais non limitativement, une colonne ayant les spécifications suivantes :
- nombre d'étages théoriques : de 1 à 20, de préférence de 5 à 10,
- taux de reflux R compris entre 0 et 10 , de préférence entre 1 et 5.

En bas de colonne, on récupère un culot de distillation comprenant l'acide difluoroacétique et en tête de colonne une phase gazeuse constituée par l'ester de formule (III).

On refroidit cette phase gazeuse et la transforme sous forme liquide par refroidissement à une température par exemple comprise entre -10°C et 25°C, de préférence comprise entre 0°C et 10 °C.

Cette opération est conduite par passage dans un condenseur qui est un appareil classique par exemple un échangeur tubulaire alimenté par de l'eau ou par un fluide maintenu à une température voisine de la température de refroidissement choisie.

Le flux condensé est introduit, latéralement en tête de colonne de distillation afin d'assurer le reflux de la colonne.

On récupère en pied de distillation, l'acide difluoroacétique avec une très bonne pureté chimique.

Dans le cas où il est mis en oeuvre un catalyseur transestérification, celui se retrouve également dans le pied de distillation et il y a lieu de séparer l'acide difluoroacétique à partir de ce milieu par une opération de distillation supplémentaire à une température de 132°C-133°C lorsque la distillation est conduite sous pression atmosphérique.

Un autre mode d'exécution de l'invention consiste à mettre en oeuvre le procédé de l'invention en continu et de faire une distillation réactive ce qui signifie que l'on effectue le mélange et la distillation dans une colonne de distillation.

Ce mode de réalisation de l'invention est préféré.

On alimente en continu la colonne de distillation à l'aide des deux réactifs, à savoir l'ester de l'acide difluoroacétique de formule (I) et l'acide aliphatique carboxylique de formule (II).

Plus précisément, l'alimentation de l'ester de l'acide difluoroacétique est faite de préférence dans la partie inférieure de la colonne.

L'alimentation de l'acide aliphatique carboxylique de formule (II) est faite de préférence dans la partie supérieure de la colonne.

La zone de réaction se situe de préférence à mi-hauteur de la colonne.

Conformément au procédé de l'invention, on distille l'ester de formule (III) au fur et à mesure de sa formation et l'on récupère en pied de colonne, l'acide difluoroacétique.

Le reflux de la colonne est assuré par le flux liquide récupéré suite à la condensation de l'ester de formule (III) et renvoyé latéralement sur la colonne.

Toutes les conditions de distillation, notamment température et pression sont telles que précédemment mentionnées pour le mode discontinu.

On soutire en pied de colonne, l'acide difluoroacétique formé.

Dans le cas où un catalyseur de transestérification est mis en oeuvre, celui-ci est introduit sous forme solide (résines ou acide supporté) dans la partie réactive de la colonne à distiller.

L'acide difluoroacétique est récupéré en pied de colonne et une séparation du catalyseur n'est donc pas nécessaire.

Le procédé de l'invention est avantageusement conduit dans un appareillage susceptible de résister à la corrosion provoquée par l'acide difluoroacétique.

A cet effet, on choisit des matériaux pour la partie en contact avec le milieu réactionnel résistant à la corrosion avantageusement des aciers vitrifiés ou des aciers inoxydables, tels que les aciers austénitiques [Robert H. Perry et al, Perry's Chemical Engineers' Handbook, Sixth Edition (1984), page 23-44]. et plus particulièrement les aciers inoxydables 304, 304 L, 316 ou 316 L.

On met en oeuvre un acier ayant une teneur en nickel au plus de 22 % en masse, de préférence comprise entre 6 et 20 %, et plus préférentiellement comprise entre 8 et 14 %.

Les aciers 304 et 304 L ont une teneur en nickel variant entre 8 et 12 % et les aciers 316 et 316 L ont une teneur en nickel variant entre 10 et 14 %.

On fait appel plus particulièrement aux aciers 316 L.

L'ensemble des différentes étapes du procédé de l'invention peuvent donc être mises en oeuvre en continu ou en discontinu.

On donne ci-après des exemples de réalisation de l'invention. Ces exemples sont donnés à titre illustratif et sans caractère limitatif.

Dans les exemples, on définit le taux de conversion et le rendement obtenu.

Le taux de conversion (TT) correspond au rapport entre le nombre de moles de substrat [ester de l'acide difluoroacétique de formule (I)] transformées et le nombre de moles de substrat [ester de l'acide difluoroacétique de formule (I)] engagées.

Le rendement (RR) correspond au rapport entre le nombre de moles de produit formées (l'acide difluoroacétique) et le nombre de moles de substrat [ester de l'acide difluoroacétique de formule (I)] engagées.

Les différentes analyses sont effectuées par RMN¹H et RMN¹⁹F.

### Exemple 1

Dans un réacteur en verre surmonté d'une colonne à distiller, le difluoroacétate d'éthyle (15 g ; 0,121 mol) est mis en présence d'acide formique (9 g ; 0,196 mol ; 1,6 équivalents) et de 0,1 g d'acide sulfurique à 98 % en poids.

Le milieu réactionnel est porté à la température de 70°C, sous pression atmosphérique.

Un reflux apparaît et le reflux total est maintenu jusqu'à ce que la température en tête de colonne soit stablilisé à 55°C.

Une fraction est alors recueillie de façon à maintenir la température en tête de colonne constante à 55°C.

On obtient une fraction de 9 g de formiate d'éthyle.

L'analyse du milieu réactionnel montre que l'intégralité du difluoroacétate d'éthyle a été transformé en acide difluoroacétique (TT = 100%).

L'acide difluoroacétique obtenu a une pureté supérieure à 98 % en poids.

Le rendement (RR) est de 95 %.

### Exemple 2

Dans un réacteur en verre surmonté d'une colonne à distiller, le difluoroacétate d'éthyle (20 g ; 0,161 mol) est mis en présence d'acide formique (7,4 g ; 0,161 mol ; 1 équivalent) et de 0,1 g d'acide sulfurique à 98 % en poids.

Le milieu réactionnel est porté à la température de 70°C, sous pression atmosphérique et une fraction de distillation est alors recueillie de façon à maintenir la température en tête de colonne constante à 55°C.

On obtient une fraction de 12 g de formiate d'éthyle.

L'analyse du milieu réactionnel montre que l'intégralité du difluoroacétate d'éthyle a été transformé en acide difluoroacétique (TT = 100 %).

Le milieu est alors distillé sous pression atmosphérique.

On récupère une fraction (m = 14,8 g) ayant un point d'ébullition de 133°C.

L'acide difluoroacétique obtenu a une pureté supérieure à 98 % en poids.

Le rendement (RR) est de 96 %.

### Exemple 3

Dans un réacteur en verre surmonté d'une colonne à distiller, le difluoroacétate d'éthyle (60 g ; 0,484 mol) est mis en présence d'acide trifluoroacétique (56 g ; 0,484 mol ; 1 équivalent).

Le milieu réactionnel est porté à la température de 85°C, sous pression atmosphérique.

Le trifluoroacétate d'éthyle est distillé en maintenant la température en tête de distillation de 63°C.

On obtient une fraction de 69 g de trifluoroacétate d'éthyle.

Le difluoroacétate d'éthyle a été intégralement consommé (TT = 100 %) et l'acide difluoroacétique obtenu en pied de distillation a une pureté de 99,5 % en poids.

Le rendement (RR) est supérieur à 99 %.

## Revendications

1. Procédé de préparation de l'acide difluoroacétique **caractérisé par le fait qu'**il comprend :
- la réaction d'un ester de l'acide difluoroacétique avec un acide aliphatique carboxylique conduisant, suite à une réaction de transestérification, à la formation de l'acide difluoroacétique et de l'ester de l'acide carboxylique correspondant ; l'acide carboxylique étant choisi de telle sorte que l'ester dudit acide carboxylique ait un point d'ébullition inférieur à celui de l'acide difluoroacétique,
- l'élimination par distillation de l'ester dudit acide carboxylique au fur et à mesure de sa formation permettant ainsi de récupérer l'acide difluoroacétique.

2. Procédé selon la revendication 1 **caractérisé par le fait que** l'ester de l'acide difiluoroacétique répondent à la formule suivante :
H-CF₂-COOR₁ (I)
dans ladite formule :
- R₁ représente un groupe alkyle ayant de 1 à 4 atomes de carbone,.

3. Procédé selon la revendication 2 **caractérisé par le fait que** l'ester de l'acide difluoroacétique est l'ester méthylique ou éthylique de l'acide difluoroacétique.

4. Procédé selon la revendication 1 **caractérisé par le fait que** l'acide aliphatique carboxylique répond à la formule suivante :
R₂-COOH (II)
dans ladite formule :
- R₂ représente un atome d'hydrogène, un groupe alkyle ayant 1 ou 2 atomes de carbone, un groupe halogénoalkyle ayant 1 atome de carbone dont un à 3 atomes d'hydrogène peuvent être remplacés par un atome d'halogène.

5. Procédé selon la revendication 4 **caractérisé par le fait que** l'acide aliphatique carboxylique est l'acide formique, l'acide acétique, l'acide trifluoroacétique, l'acide monochloroacétique, l'acide trichloroacétique.

6. Procédé selon la revendication 1 **caractérisé par le fait que** l'on fait réagir l'ester de l'acide difluoroacétique de formule (I) et l'acide aliphatique carboxylique de formule (II), dans des proportions telles que le rapport entre le nombre de moles d'ester d'acide difluoroacétique et le nombre de moles de l'acide aliphatique carboxylique varie entre 0,8 et 1,2.

7. Procédé selon la revendication 1 **caractérisé par le fait que** la réaction de transestérification est conduite en présence d'un catalyseur de type acide fort.

8. Procédé selon l'une des revendications 1 à 7 **caractérisé par le fait qu'**il comprend au moins une étape de mélange de l'ester de l'acide difluoroacétique de formule (I) et de l'acide aliphatique carboxylique de formule (II) et une étape de distillation pour obtenir
- en pied de distillation, l'acide difluoroacétique attendu,
- et en tête de distillation, une phase gazeuse comprenant l'ester formé de formule (III).

9. Procédé selon la revendication 8 **caractérisé par le fait qu'**il comprend comprend une opération de mélange de l'ester de l'acide difluoroacétique de formule (I) et de l'acide aliphatique carboxylique de formule (II) par mise en contact des deux réactifs puis l'on porte le mélange réactionnel maintenu sous pression atmosphérique, à une température telle que l'on distille l'ester de l'acide carboxylique de formule (III) au fur et à mesure de sa formation.

10. Procédé selon l'un des revendications 8 et 9 **caractérisé par le fait que** l'on introduit les réactifs séparément ou en mélange dans un réacteur équipé de moyens d'agitation et de chauffage et surmonté d'une colonne de distillation.

11. Procédé selon l'un des revendications 1 à 7 **caractérisé par le fait que** l'on effectue une distillation réactive.

12. Procédé selon la revendication 11 **caractérisé par le fait que** l'on alimente en continu la colonne de distillation à l'aide des deux réactifs, à savoir l'ester de l'acide difluoroacétique de formule (I) et l'acide aliphatique carboxylique de formule (II).

13. Procédé selon l'un des revendications 8 à 12 **caractérisé par le fait que** le reflux de la colonne est assuré par le flux liquide récupéré suite à la condensation de l'ester de formule (III) et renvoyé latéralement sur la colonne.

## Patentansprüche

1. Verfahren zur Herstellung von Difluoressigsäure, **dadurch gekennzeichnet, dass** es Folgendes umfasst:
- Umsetzen eines Difluoressigsäureesters mit einer aliphatischen Carbonsäure, was nach einer Umesterungsreaktion zur Bildung von Difluor essig säure und dem entsprechenden Carbonsäure ester führt; wobei die Carbonsäure so gewählt wird, dass der Ester der Carbonsäure einen Siede punkt aufweist, der unter dem Siedepunkt von Difluoressigsäure liegt,
- Abdestillieren des Esters der Carbonsäure so schnell, wie er gebildet wird, was die Gewinnung der Difluoressigsäure ermöglicht.

2. Verfahren nach Anspruch 1, **dadurch gekennzeichnet, dass** der Difluoressigsäureester die folgende Formel aufweist:
H-CF₂-COOR₁ (I)
worin
- R¹ für eine Alkylgruppe mit 1 bis 4 Kohlen stoff atomen steht.

3. Verfahren nach Anspruch 2, **dadurch gekennzeichnet, dass** es sich bei dem Difluoressigsäureester um Difluoressigsäuremethylester oder -ethylester handelt.

4. Verfahren nach Anspruch 1, **dadurch gekennzeichnet, dass** die aliphatische Carbonsäure die folgende Formel aufweist:
R₂-COOH (II)
worin
- R² für ein Wasserstoffatom, eine Alkylgruppe mit 1 oder 2 Kohlenstoffatomen oder eine Halogenalkylgruppe mit 1 Kohlenstoffatom, wobei 1 bis 3 Wasserstoff atome durch ein Halogenatom ersetzt sein können, steht.

5. Verfahren nach Anspruch 4, **dadurch gekennzeichnet, dass** es sich bei der aliphatischen Carbonsäure um Ameisensäure, Essigsäure, Trifluoressigsäure, Mono-chloressigsäure oder Trichloressigsäure handelt.

6. Verfahren nach Anspruch 1, **dadurch gekennzeichnet, dass** der Difluoressigsäureester der Formel (I) und die aliphatische Carbonsäure der Formel (II) in solchen Proportionen umgesetzt werden, dass das Verhältnis zwischen der Zahl der Mole von Difluoressig säure ester und der Zahl der Mole der aliphatischen Carbonsäure zwischen 0,8 und 1,2 variiert.

7. Verfahren nach Anspruch 1, **dadurch gekennzeichnet, dass** die Umesterungsreaktion in Gegenwart eines Katalysators vom Typ starke Säure durchgeführt wird.

8. Verfahren nach einem der Ansprüche 1 bis 7, **dadurch gekennzeichnet, dass** es mindestens einen Schritt des Mischens des Difluoressigsäureesters der Formel (I) und der aliphatischen Carbonsäure der Formel (II) und einen Destillationsschritt zum Erhalt
- der erwarteten Difluoressigsäure am Sumpf der Destillation
- und einer Gasphase, die den gebildeten Ester der Formel (III) umfasst, am Kopf der Destillation
umfasst.

9. Verfahren nach Anspruch 8, **dadurch gekennzeichnet, dass** es einen Arbeitsgang des Mischens des Difluoressigsäureesters der Formel (I) und der aliphatischen Carbonsäure der Formel (II) durch Inberührungbringen der beiden Reaktanten umfasst und man das erhaltene, unter Atmosphärendruck gehalte ne Reaktionsgemisch auf eine solche Temperatur bringt, dass der Carbonsäureester der Formel (III) so schnell abdestilliert wird, wie er gebildet wird.

10. Verfahren nach einem der Ansprüche 8 und 9, **dadurch gekennzeichnet, dass** die Reaktanten separat oder als Mischung in einen Reaktor mit Rühr- und Heizeinrichtungen und aufgesetzter Destillationskolonne eingetragen werden.

11. Verfahren nach einem der Ansprüche 1 bis 7, **dadurch gekennzeichnet, dass** eine Reaktiv destillation durchgeführt wird.

12. Verfahren nach Anspruch 11, **dadurch gekennzeichnet, dass** der Destillationskolonne die beiden Reaktanten, nämlich der Difluor essig säure ester der Formel (I) und die aliphatische Carbonsäure der Formel (II), kontinuierlich zugeführt werden.

13. Verfahren nach einem der Ansprüche 8 bis 12, **dadurch gekennzeichnet, dass** der Rücklauf der Kolonne durch den nach der Kondensation des Esters der Formel (III) gewonnenen und seitlich in die Kolonne zurückgeführten Flüssigkeitsstrom gewährleistet wird.

## Claims

1. Process for the preparation of difluoro acetic acid, **characterized in that** it comprises:
- the reaction of a difluoroacetic acid ester with an aliphatic carboxylic acid, resulting, subsequent to a transesterification reaction, in the formation of difluoroacetic acid and the corresponding carboxylic acid ester, the carboxylic acid being chosen so that the ester of said carboxylic acid has a boiling point below that of difluoroacetic acid,
- the removal by distillation of the ester of said carboxylic acid as it is formed, thus making it possible to recover the difluoroacetic acid.

2. Process according to Claim 1, **characterized in that** the difluoroacetic acid ester corresponds to the following formula:
H-CF₂-COOR₁ (I)
in said formula:
- R₁ represents an alkyl group having from 1 to 4 carbon atoms.

3. Process according to Claim 2, **characterized in that** the difluoroacetic acid ester is the methyl or ethyl ester of difluoroacetic acid.

4. Process according to Claim 1, **characterized in that** the aliphatic carboxylic acid corresponds to the following formula:
R₂-COOH (II)
in said formula:
- R₂ represents a hydrogen atom, an alkyl group having 1 or 2 carbon atoms or a haloalkyl group having 1 carbon atom, 1 to 3 hydrogen atoms of which can be replaced by a halogen atom.

5. Process according to Claim 4, **characterized in that** the aliphatic carboxylic acid is formic acid, acetic acid, trifluoroacetic acid, monochloroacetic acid or trichloroacetic acid.

6. Process according to Claim 1, **characterized in that** the difluoroacetic acid ester of formula (I) and the aliphatic carboxylic acid of formula (II) are reacted in proportions such that the ratio of the number of moles of difluoroacetic acid ester to the number of moles of aliphatic carboxylic acid varies between 0.8 and 1.2.

7. Process according to Claim 1, **characterized in that** the transesterification reaction is carried out in the presence of a catalyst of strong acid type.

8. Process according to one of Claims 1 to 7, **characterized in that** it comprises at least one stage of mixing the difluoroacetic acid ester of formula (I) and the aliphatic carboxylic acid of formula (II) and one distillation stage, in order to obtain:
- at the distillation bottom, the expected difluoro-acetic acid,
- and, at the distillation top, a gas phase comprising the ester of formula (III) formed.

9. Process according to Claim 8, **characterized in that** it comprises an operation in which the difluoroacetic acid ester of formula (I) and the aliphatic carboxylic acid of formula (II) are mixed by bringing the two reactants into contact and then the reaction mixture, kept at atmospheric pressure, is brought to a temperature such that the carboxylic acid ester of formula (III) is distilled off as it is formed.

10. Process according to either of Claims 8 and 9, **characterized in that** the reactants are introduced, separately or as a mixture, into a reactor equipped with stirring and heating means and surmounted by a distillation column.

11. Process according to one of Claims 1 to 7, **characterized in that** a reactive distillation is carried out.

12. Process according to Claim 11, **characterized in that** the distillation column is fed continuously using the two reactants, namely the difluoroacetic acid ester of formula (I) and the aliphatic carboxylic acid of formula (II).

13. The process according to one of Claims 8 to 12, **characterized in that** the reflux of the column is provided by the liquid stream recovered subsequent to the condensation of the ester of formula (III) and returned laterally to the column.
